# EUROPEAN PATENT SPECIFICATION

(11) **EP 1 731 498 B1**
(45) Date of publication and mention of the grant of the patent: **19.05.2010**
(21) Application number: 05720669.0
(22) Date of filing: 08.03.2005
(51) Int. Cl.: C07C 67/08, B01J 31/02, C07C 69/612, C07C 69/708, C07C 69/78, C07B 61/00

(54) **METHOD FOR PRODUCING ESTER AND ESTERIFICATION CATALYST**
VERFAHREN ZUR HERSTELLUNG VON ESTERN UND VERESTERUNGSKATALYSATOR
PROCEDE POUR PRODUIRE UN ESTER ET CATALYSEUR D' ESTERIFICATION

(30) Priority: 08.03.2004 JP 2004063929
(43) Date of publication of application: 13.12.2006
(73) Proprietor: Nagoya Industrial Science Research Institute, Nagoya-shi, Aichi 460-0008 (JP)
(72) Inventor: ISHIHARA, Kazuaki, Konann-shi, Aichi 483-8012 (JP); SAKAKURA, Akira, No.302 Tokanmanshon Hoshigaoka, Nagoya-shi, Aichi 464-0802 (JP)
(74) Representative: Cloughley, Peter Andrew
(86) International application number: PCT/JP2005/004398
(87) International publication number: WO 2005/085172

(56) References cited:
- GACEM, BADRA ET AL: "Esterification of sterically hindered acids and alcohols in fluorous media" TETRAHEDRON LETTERS , 44(7), 1391-1393 CODEN: TELEAY; ISSN: 0040-4039, 2003, XP004405228
- DATABASE WPI Week 200407 Derwent Publications Ltd., London, GB; AN 2004-065019 XP002468153 & JP 2003 155271 A (DOKURITSU GYOSEI HOJIN KAGAKU GIJUTSU SH) 27 May 2003 (2003-05-27)
- NAKAGAWA A ET AL: 'Ammonium-en Shokubai o Mochiiru Dassui Shukugo Hanno ni yoru Ester Gosei.' THE CHEMICAL SOCIETY OF JAPAN. 11 March 2004, page 1265, XP002996616
- WAKASUGI K ET AL: 'Diphenylammonium triflate (DPAT): efficient catalyst for esterification of carboxylic acids and for transesterification of carboxylic esters with nearly equimolar amounts of alcohols.' TETRAHEDRON LETTERS. vol. 41, 2000, pages 5249 - 5252, XP004222048

## Description

### Technical Field

The present invention relates to processes for producing esters and esterification catalysts. More particularly, the invention relates to a process for producing an ester by a reaction between a carboxylic acid and an alcohol, and an esterification catalyst used for the process.

### Background Art

Esterification is the most fundamental and important reaction in organic synthesis. There have been an enormous number of reports on esterification reactions. Recently, the present inventors have established a process for producing an ester in high yield, in which an esterification reaction between a carboxylic acid and an alcohol is carried out using a tetravalent hafnium compound as an esterification catalyst, as shown in Japanese Unexamined Patent Application Publication No. 2002-121170. Meanwhile, there is a report in Tetrahedron Letters Vol. 41 (2000) pp. 5249-5252 that diphenylammonium triflate promotes an esterification reaction between a carboxylic acid and an alcohol under mild conditions. The diphenylammonium triflate is considered to be an interesting esterification catalyst from the standpoint that it does not contain a metal.

### Disclosure of Invention

The present inventors have studied and found that diphenylammonium triflate has lower catalytic activity than tetravalent hafnium compounds, and when a secondary alcohol is used as a substrate, a side reaction occurs with the formation of an olefin.

The present invention has been achieved to overcome the problems described above. It is an object of the present invention to provide a novel process for producing an ester in which esterification proceeds rapidly and even in the case of a reaction substrate that can form an olefin as a by-product, it is possible to effectively suppress the by-production of the olefin. It is another object of the present invention to provide a novel esterification catalyst which has high catalytic activity for esterification and which can effectively suppress the by-production of an olefin even in the case of a reaction substrate that can form an olefin as a by-product.

In order to overcome the problems described above, the present inventors have conducted intensive research on a novel esterification catalyst superior to diphenylammonium triflate and, as a result, have found that an ammonium sulfonate composed of an ammonium cation having a certain basic skeleton and a certain sulfonic acid anion, has high catalytic activity and can effectively suppress the by-production of an olefin even in the case of a reaction substrate that can form an olefin as a by-product. Thus, the present invention has been completed.

That is, the present invention relates to a process for producing an ester as set forth in claim 7, in which an esterification catalyst as set forth in claim 1 is used, involving formula (1) below.

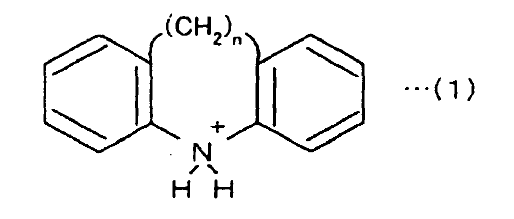

If the carboxylic acid is allowed to react with the alcohol using the ammonium sulfonate described in claim 1 as the esterification catalyst, esterification proceeds rapidly compared with the case where known diphenylammonium triflate is used as the esterification catalyst, and even in the case of a reaction substrate that can form an olefin as a by-product, it is possible to effectively suppress the by-production of the olefin. Here, the olefin is formed mainly by elimination of the hydroxyl group of the alcohol or elimination of the acyl group of the ester.

In the ammonium cation of formula (1), hydrogen atoms in the benzene rings and hydrogen atoms bonded to bridge carbon may not be substituted, or hydrogen atoms in the benzene rings and hydrogen atoms bonded to bridge carbon may be partially or wholly substituted. In the latter case, preferably, the hydrogen atoms at the ortho positions (the carbon atoms adjacent to the carbon atoms bonded to nitrogen) in the benzene rings are substituted. Examples of the substituent include an alkyl group, an alkenyl group, a cycloalkyl group, an aryl group, an alkoxy group, a nitro group, a cyano group, and a halogen. Among these, examples of the alkyl group include linear or branched alkyl groups having 1 to 10 carbon atoms, such as a methyl group, an ethyl group, an n-propyl group, an isopropyl group, an n-butyl group, a sec-butyl group, and a tert-butyl group.

The ammonium cation of formula (1) may be iminodibenzyl in which n represents 2 and hydrogen atoms in the benzene rings and hydrogen atoms bonded to bridge carbon are not substituted. The iminodibenzyl is easily available, has high catalytic activity for esterification, and also has a high effect of suppressing olefination, which is a side reaction.

The present invention also relates to a process for producing an ester as set forth in claim 7, in which an esterification catalyst as set forth in claim 4 is used, involving formula (2) below.

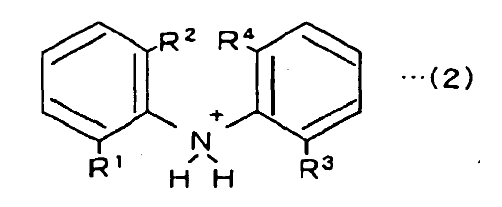

If the carboxylic acid is allowed to react with the alcohol using the ammonium sulfonate described in claim 4 as the esterification catalyst, esterification proceeds rapidly compared with the case where known diphenylammonium triflate is used as the esterification catalyst, and even in the case of a reaction substrate that can form an olefin as a by-product, it is possible to effectively suppress the by-production of the olefin.

In the ammonium cation of formula (2), R¹ to R⁴, which may be the same or different, each represent a linear or branched alkyl group having 1 to 10 carbon atoms. Examples of such an alkyl group include a methyl group, an ethyl group, an n-propyl group, an isopropyl group, an n-butyl group, a sec-butyl group, and a tert-butyl group.

In the ammonium cation of formula (2), hydrogen atoms at the para positions (the para positions with respect to the carbon atoms bonded to nitrogen) in the benzene rings each may be substituted with a linear or branched alkyl group having 1 to 10 carbon atoms. Examples of such an alkyl group include a methyl group, an ethyl group, an n-propyl group, an isopropyl group, an n-butyl group, a sec-butyl group, and a tert-butyl group.

Examples of the sulfonic acid anion of the ammonium sulfonate used in the process for producing the ester according to the present invention include benzenesulfonic acid anions having an electron-withdrawing group at least at the ortho position, the meta position, or the para position, such as a pentafluorobenzenesulfonic acid anion and perfluoroalkanesulfonic acid anions, such as a trifluoromethanesulfonic acid anion. Among these, a pentafluorobenzenesulfonic acid anion is more preferable.

In the process for producing the ester according to the present invention, although the reaction proceeds when either one of the carboxylic acid and the alcohol is used in excess amount, the reaction also proceeds when equimolar amounts of the carboxylic acid and the alcohol are used.

The carboxylic acid used in the process for producing the ester according to the present invention is not particularly limited. Examples thereof include monocarboxylic acids including saturated fatty acids, such as acetic acid, propionic acid, butyric acid, valeric acid, caproic acid, enanthic acid, caprylic acid, pelargonic acid, capric acid, lauric acid, myristic acid, and stearic acid, and unsaturated fatty acids, such as oleic acid; dicarboxylic acids, such as oxalic acid, malonic acid, succinic acid, glutaric acid, adipic acid, pimelic acid, suberic acid, azelaic acid, sebacic acid, dodecanedioic acid, fumaric acid, maleic acid, terephthalic acid, isophthalic acid, and diphenyl ether-4,4'-dicarboxylic acid; tricarboxylic acids, such as butane-1,2,4-tricarboxylic acid, cyclohexane-1,2,3-tricarboxylic acid, benzene-1,2,4-tricarboxylic acid, and naphthalene-1,2,4-tricarboxylic acid; and tetracarboxylic acids, such as butane-1,2,3,4-tetracarboxylic acid, cyclobutane-1,2,3,4-tetracarboxylic acid, benzene-1,2,4,5-tetracarboxylic acid, 3,3',4,4'-benzophenone tetracarboxylic acid, and 3,3',4,4'-diphenyl ether tetracarboxylic acid. As necessary, these carboxylic acids may have an alkyl group, an alkenyl group, a cycloalkyl group, an aryl group, an alkoxy group, a nitro group, a cyano group, a halogen, or the like.

The alcohol used in the process for producing the ester according to the present invention is not particularly limited. Examples thereof include aliphatic monohydric alcohols, such as methanol, ethanol, propanol, butanol, pentanol, hexanol, heptanol, octanol, decanol, undecanol, dodecanol, and stearyl alcohol; alicyclic monohydric alcohols, such as cyclohexanol and cyclododecanol; aromatic monohydric alcohols, such as benzyl alcohol; polyhydric alcohols, such as ethylene glycol, propylene glycol, neopentyl glycol, trimethylol propane, trimethylol ethane, pentaerythritol, dipentaerythritol, sorbitol, and polyvinyl alcohol; and alicyclic dihydric alcohols, such as cyclohexane-1,2-diol and cyclopentane-1,2-diol. These alcohols may be primary alcohols or secondary alcohols. When a secondary alcohol is used, an olefin may be formed. However, it is possible to suppress the formation of the olefin compared with using known diphenylammonium triflate. Furthermore, it is possible to use an alcohol, such as benzyl alcohol or allyl alcohol, that is susceptible to acids. It is also possible to use 1,2-diol that cannot be used with a Lewis acid metal, such as a hafnium (IV) salt or a zirconium (IV) salt.

The reaction solvent used in the process for producing the ester according to the present invention is not particularly limited as long as it is inactive in the esterification reaction. The reaction solvent may be a nonpolar solvent or a polar solvent. Examples of the reaction solvent include alkanes, such as hexane, heptane, and octane; haloalkanes, such as methylene chloride, chloroform, carbon tetrachloride, and ethylene chloride; benzenes, such as benzene, toluene, xylene, mesitylene, and pentamethylbenzene; halobenzenes, such as chlorobenzene and bromobenzene; and ethers, such as diethyl ether and anisole. Furthermore, in the process for producing the ester according to the present invention, it is not always necessary to use the reaction solvent, and the reaction also proceeds in the absence of a solvent.

The amount of the ammonium sulfonate used in the process for producing the ester according to the present invention is appropriately set depending on the reaction system (types of carboxylic acid and alcohol, type of reaction solvent, etc.). For example, the ammonium sulfonate is used preferably in an amount of 0.1 to 20 mol%, in particular, 1 to 10 mol%, relative to the amount of the carboxylic acid or the alcohol whichever lower. The ammonium sulfonate is typically obtained by mixing substantially the same amounts of an amine and a sulfonic acid, followed by evaporation to dryness.

In the process for producing the ester according to the present invention, the reaction temperature and the reaction time are appropriately set depending on the reaction system. The reaction temperature is usually room temperature (e.g., 10°C to 30°C) or higher, and preferably 50°C or higher. The reaction is carried out for a period of time in which either the carboxylic acid or the alcohol substantially disappears (usually 0.5 to 72 hours). However, in order to suppress by-products (an olefin and the like), the reaction temperature is preferably set according to the alcohol or the like, which is the reaction substrate, in a range of room temperature to 90°C (more preferably 80°C). In particular, the esterification reaction in which a primary alcohol is used, the reaction proceeds under the conditions of absence of a solvent and room temperature. In addition, it is not always necessary to actively remove, from the reaction system, water produced in the esterification reaction.

### Brief Description of the Drawings

Fig. 1 is a graph showing the experimental results in Comparative Example 1.
Fig. 2 is a graph showing the experimental results in Comparative Example 2.
Fig. 3 is a graph showing the experimental results in Comparative Example 3.
Fig. 4 is a graph showing the experimental results in Example 1.
Fig. 5 is a graph showing the experimental results in Example 2.
Fig. 6 is a graph showing the experimental results in Example 3.
Fig. 7 is a graph showing the experimental results in Example 4.
Fig. 8 is a graph showing the experimental results in Example 5.
Fig. 9 is a graph showing the experimental results in Example 6.
Fig. 10 is a table showing the experimental results in Examples 2 and 7 to 11 and Comparative Examples 1 and 4 to 8.
Fig. 11 is a table showing the experimental results in Examples 12 to 21.
Fig. 12 is a table showing the experimental results in Examples 22 to 27.

### Best Mode for Carrying Out the Invention

### [Measurement equipment and devices]

As a nuclear magnetic resonance (NMR) device, a Gemini 2000 (¹HNMR 300 MHz) manufactured by Varian, Inc. was used. In silica gel thin-layer chromatography (TLC) for analysis and for preparation, silica gel 60GF254 (thickness 0.25 nm) manufactured by Merck was used. In preparatory column chromatography, silica gel grade 9385 manufactured by Merck was used.

### [General production methods]

### (1) Production method for ammonium sulfonate

An amine (1.0 mmol) and a sulfonic acid (1.0 mmol) were placed in a flask and dissolved in toluene (2 ml) at room temperature. The resulting mixture was stirred for 30 minutes. The product was subjected to evaporation drying and washing with hexane (5 ml) three times to give a purified product.

### (2) Production method for carboxylate ester

A carboxylic acid (1.0 mmol), an alcohol (1.0 mmol), and an ammonium salt (1 to 10 mol%) were subjected to reflux under heating in heptane (2 ml) at 115°C (temperature of oil bath), and the reaction mixture was stirred until the alcohol was completely consumed over TLC. Under reduced pressure, heptane was evaporated to dryness to obtain a crude product. The crude product was purified by column chromatography (hexane:ethyl acetate = 30:1) to give the desired carboxylate ester.

### [Comparative Example 1]

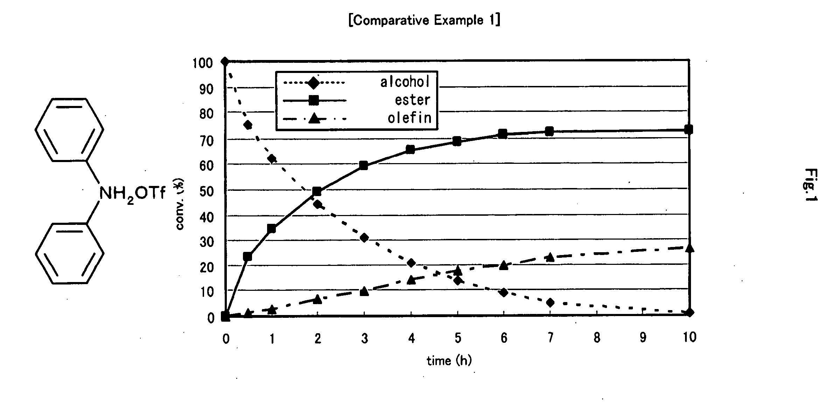

Using known diphenylammonium triflate as the esterification catalyst, 4-phenylbutyric acid as the carboxylic acid, and cyclododecanol as the alcohol, cyclododecyl 4-phenylbutyrate was obtained according to the general production method (2) described above (refer to formula (3) below). The esterification catalyst was used in an amount of 5 mol% relative to the amount of the carboxylic acid. In this reaction, cyclododecene (olefin) was formed as the by-product. Fig. 1 is a graph which shows the changes in the alcohol, the ester, and the olefin with time in this reaction. As is evident from Fig. 1, after 10 hours, the conversion ratio of the carboxylate ester was 73%, and the conversion ratio of the olefin was 27%. These conversion ratios were relative to the amount of the alcohol, and were obtained from the peak areas in ¹H-NMR.

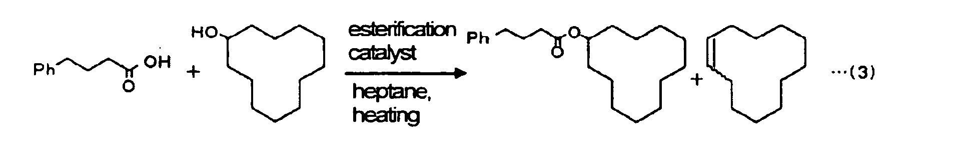

### [Comparative Example 2]

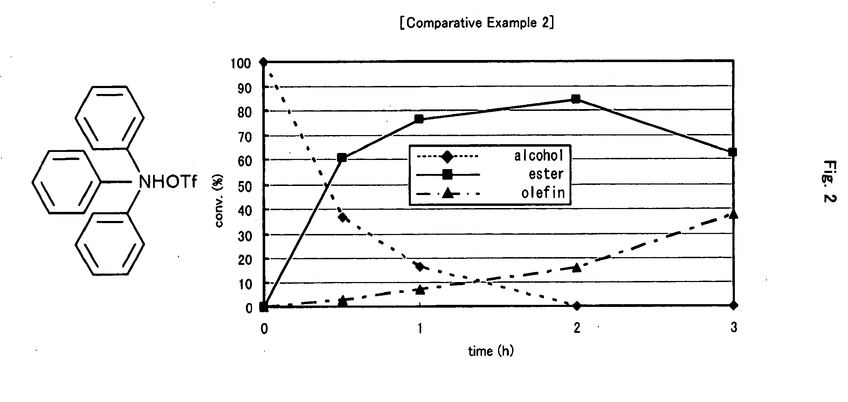

Using triphenylamine as the amine and trifluoromethanesulfonic acid as the sulfonic acid, triphenylammonium triflate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. Fig. 2 is a graph which shows the changes in the alcohol, the ester, and the olefin with time in this reaction. As is evident from Fig. 2, after 2 hours, the conversion ratio of the carboxylate ester reached 84%. However, after 3 hours, the conversion ratio of the carboxylate ester decreased to 62%, and instead, the conversion ratio of the olefin increased to 38%. In this example, since the alcohol disappeared after 2 hours, the olefin was considered to be formed by deacylation of the carboxylate ester. Consequently, it is known that although the triphenylammonium salt has higher catalytic activity than the diphenylammonium salt in Comparative Example 1, the olefin is easily formed, and thus it is difficult to control the esterification.

### [Comparative Example 3]

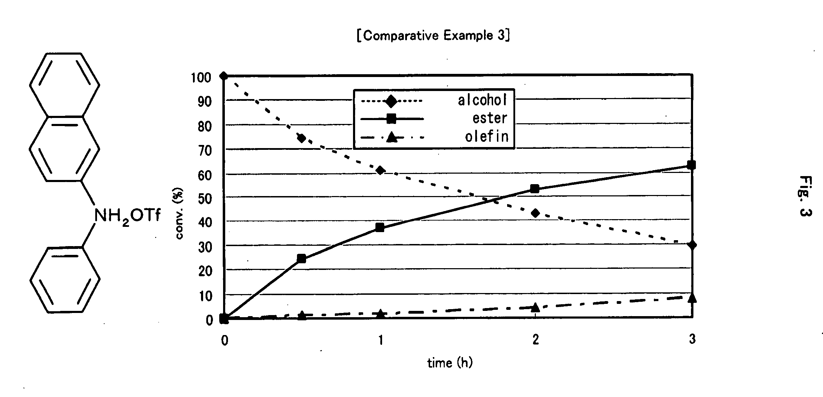

Using 2-naphthylphenylamine as the amine and trifluoromethanesulfonic acid as the sulfonic acid, 2-naphthylphenylammonium triflate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. Fig. 3 is a graph which shows the changes in the alcohol, the ester, and the olefin with time in this reaction. As is evident from Fig. 3, after 3 hours, the conversion ratio of the carboxylate ester was 63%, and the conversion ratio of the olefin was 29%. Thus, it is known that it is difficult to suppress the formation of the olefin.

### [Example 1]

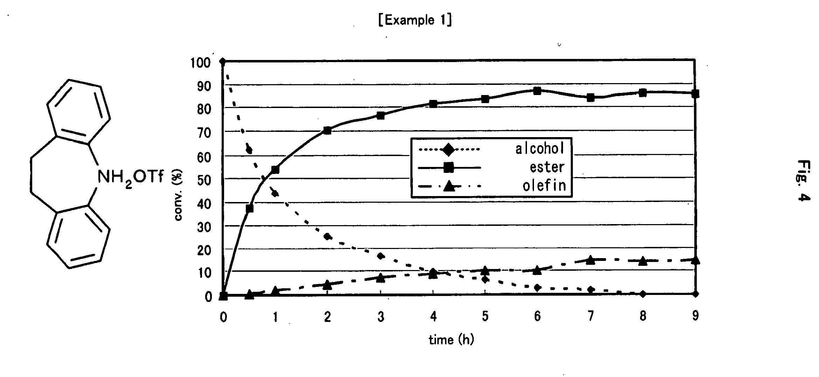

Using iminodibenzyl as the amine and trifluoromethanesulfonic acid as the sulfonic acid, iminodibenzyl triflate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. Fig. 4 is a graph which shows the changes in the alcohol, the ester, and the olefin with time in this reaction. As is evident from Fig. 4, after 8 hours, the conversion ratio of the carboxylate ester was 86%, and the conversion ratio of the olefin was 14%. Thereafter until 9 hours, the ratios remained substantially level. Thus, it is known that iminodibenzyl triflate has higher catalytic activity for esterification than the diphenylammonium triflate in Comparative Example 1, and moreover effectively suppresses the formation of the olefin.

### [Example 2]

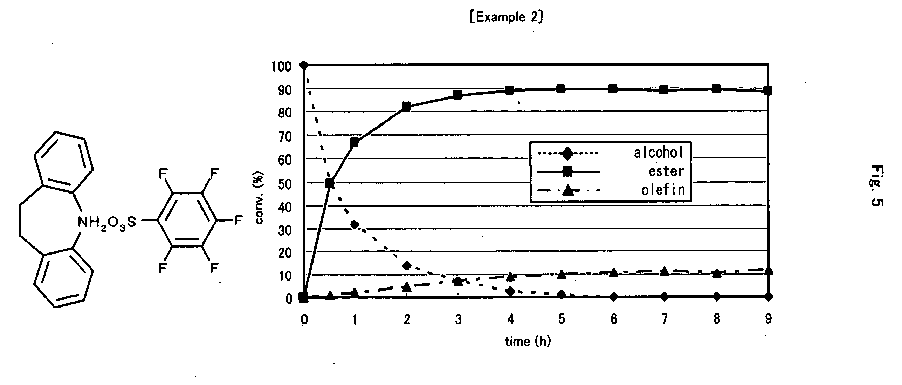

Using iminodibenzyl as the amine and pentafluorobenzenesulfonic acid as the sulfonic acid, iminodibenzyl pentafluorobenzenesulfonate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. Fig. 5 is a graph which shows the changes in the alcohol, the ester, and the olefin with time in this reaction. As is evident from Fig. 5, after 6 hours, the conversion ratio of the carboxylate ester was 90%, and the conversion ratio of the olefin was 10%. Thereafter until 9 hours, the ratios remained substantially level. Thus, it is known that iminodibenzyl pentafluorobenzenesulfonate has higher catalytic activity for esterification than the diphenylammonium triflate in Comparative Example 1, and moreover effectively suppresses the formation of the olefin.

### [Example 3]

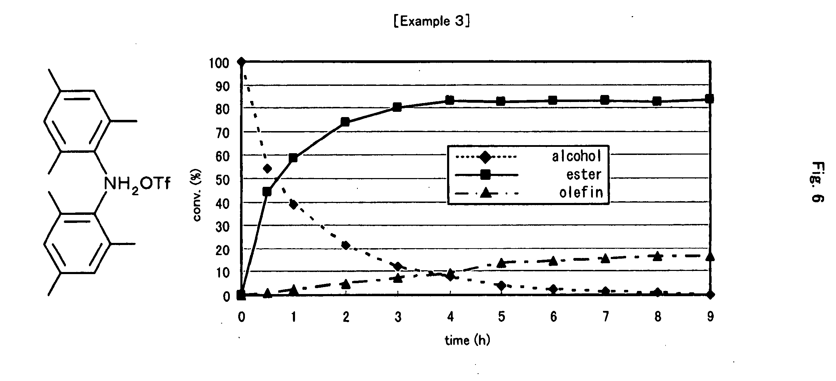

Using dimesitylamine as the amine and trifluoromethanesulfonic acid as the sulfonic acid, dimesitylammonium triflate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. The dimesitylamine was synthesized according to literature (J. Phys. Chem. A, vol. 106 (2000), pp. 11719-11725). Fig. 6 is a graph which shows the changes in the alcohol, the ester, and the olefin with time in this reaction. As is evident from Fig. 6, after 9 hours, the conversion ratio of the carboxylate ester was 84%, and the conversion ratio of the olefin was 16%. Thus, it is known that dimesitylammonium triflate has higher catalytic activity for esterification than the diphenylammonium triflate in Comparative Example 1, and moreover effectively suppresses the formation of the olefin.

### [Example 4]

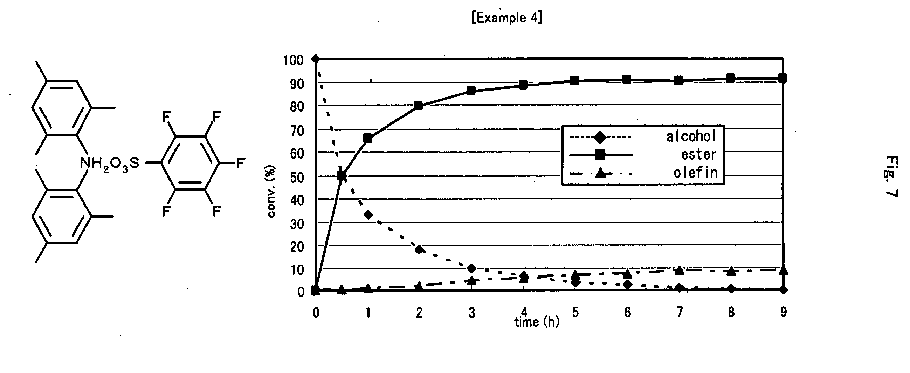

Using dimesitylamine as the amine and pentafluorobenzenesulfonic acid as the sulfonic acid, dimesitylammonium pentafluorobenzenesulfonate was obtained according to the general production method (1) described above. Using this salt as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid. Fig. 7 is a graph which shows the changes in the alcohol, the ester, and the olefin with time in this reaction. As is evident from Fig. 7, after 9 hours, the conversion ratio of the carboxylate ester was 91%, and the conversion ratio of the olefin was 9%. Thus, it is known that dimesitylammonium pentafluorobenzenesulfonate has higher catalytic activity for esterification than the diphenylammonium triflate in Comparative Example 1, and moreover effectively suppresses the formation of the olefin.

### [Example 5]

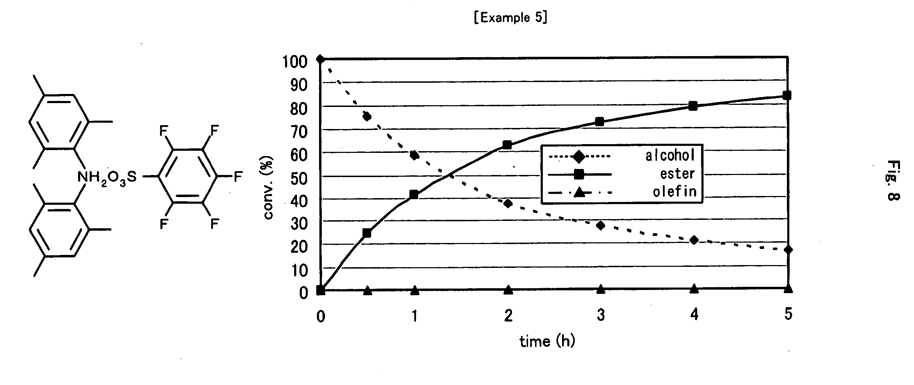

An esterification reaction was carried out as in Example 4 except that the reaction temperature was set at 80°C (temperature of oil bath). Fig. 8 is a graph which shows the changes in the alcohol, the ester, and the olefin with time in this reaction. As is evident from Fig. 8, after 5 hours, the conversion ratio of the carboxylate ester was 83%, and the conversion ratio of the olefin was 0%. Although not shown in the drawing, after 22 hours, the conversion ratio of the carboxylate ester was 97%, and the conversion ratio of the olefin was 0%. From the reaction results in Examples 4 and 5, it is known that by decreasing the reaction temperature (temperature of oil bath) from 115°C to 80°C, the formation of the olefin can be substantially completely suppressed.

### [Example 6]

Using 2,6-diisopropylphenylmesitylamine as the amine and pentafluorobenzenesulfonic acid as the sulfonic acid, 2,6-diisapropylphenylmesitylammonium pentafluorobenzenesulfonate was obtained according to the general production method (1) described above. The 2,6-diisopropylphenylmesitylamine was synthesized by performing a coupling reaction between 2,6-diisopropylaniline and 1-bromo-2,4,6-trimethylbenzene according to known literature (J. Org. Chem., 1998, vol. 63, pp. 7727-7737). The ¹H-NMR data for the resulting amine are shown below. The chemical shift is represented by δ (ppm), and tetramethylsilane (TMS) is used as an internal standard compound, the chemical shift of its signal being δ=0. The coupling constant is represented by J. With respect to the splitting patterns of signals, the singlet is abbreviated as s, the doublet is abbreviated as d, the septet is expressed as septet, and the broad singlet is abbreviated as brs. ¹H-NMR (CDCl3, 300 MHz) ; δ 1.10 (d, J=6.9 Hz, 12H), 1.95 (s, 6H), 2.22 (s, 3H), 3.12 (septet, J=6.9 Hz, 2H), 4.69 (brs, 1H), 6.76 (s, 2H), 7.10 (brs, 3H).

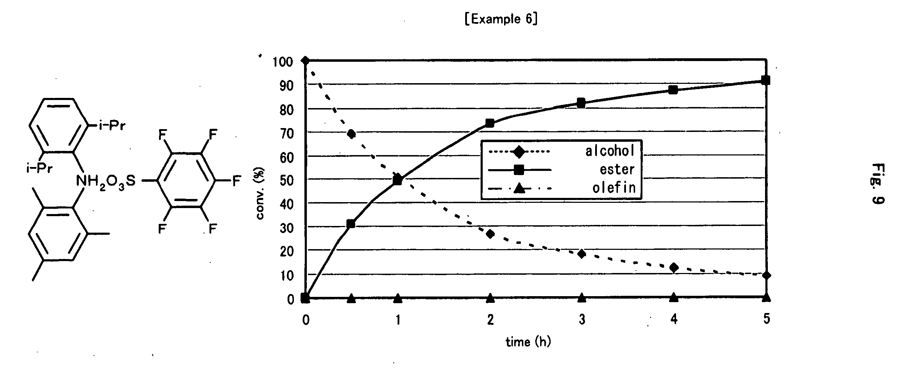

Using diisopropylphenylmesitylammonium pentafluorobenzenesulfonate as the esterification catalyst, 4-phenylbutyric acid as the carboxylic acid, and cyclododecanol as the alcohol, cyclododecyl 4-phenylbutyrate was obtained according to the general production method (2) described above (refer to formula (3)). The esterification catalyst was used in an amount of 5 mol% relative to the amount of the carboxylic acid, and the reaction temperature (temperature of oil bath) was set at 80°C. Fig. 9 is a graph which shows the changes in the alcohol, the ester, and the olefin with time in this reaction. As is evident from Fig. 9, after 9 hours, the conversion ratio of the carboxylate ester was 91%, and the conversion ratio of the olefin was 0%. Although not shown in the drawing, after 22 hours, the conversion ratio of the carboxylate ester was greater than 98%, and the conversion ratio of the olefin was less than 2%. From the reaction results in Examples 5 and 6, it is known that by setting the reaction temperature (temperature of oil bath) at 80°C, the formation of the olefin can be substantially completely suppressed, and that by changing the methyl group to the more bulky group (isopropyl group) at the two ortho positions of the aryl group (phenyl group) bonded to the amine in the esterification catalyst, esterification is promoted.

### [Examples 7 to 11 and Comparative Examples 4 to 8]

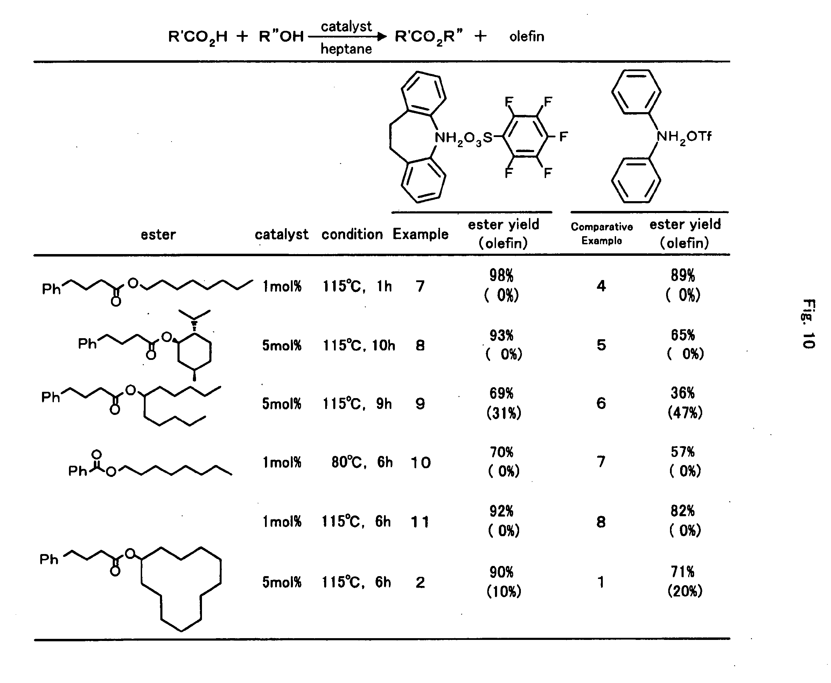

As the esterification catalyst, iminodibenzyl pentafluorobenzenesulfonate was used in each of Examples 7 to 11, and diphenylammonium triflate was used in each of Comparative Examples 4 to 8. As the carboxylic acid, 4-phenylbutyric acid was used in each of Examples 7 to 9 and Comparative Examples 4 to 6, and benzoic acid was used in each of Examples 10 and 11 and Comparative Examples 7 and 8. As the alcohol, 1-octanol was used in each of Examples 7, 10, and 11 and Comparative Examples 4, 7, and 8; 1-menthol was used in each of Example 8 and Comparative Example 5; and 6-undecanol was used in each of Example 9 and Comparative Example 6. The carboxylate esters were produced under the conditions shown in the table of Fig. 10 according to the general production method (2) described above. The results thereof are shown in the table of Fig. 10. Fig. 10 also shows the results of Example 2 and Comparative Example 1. As is evident from Fig. 10, Examples 2 and 7 to 11 exhibit higher catalytic activity for esterification than corresponding Comparative Examples 1 and 4 to 8, and moreover, effectively suppress the formation of the olefin.

### [Examples 12 to 21]

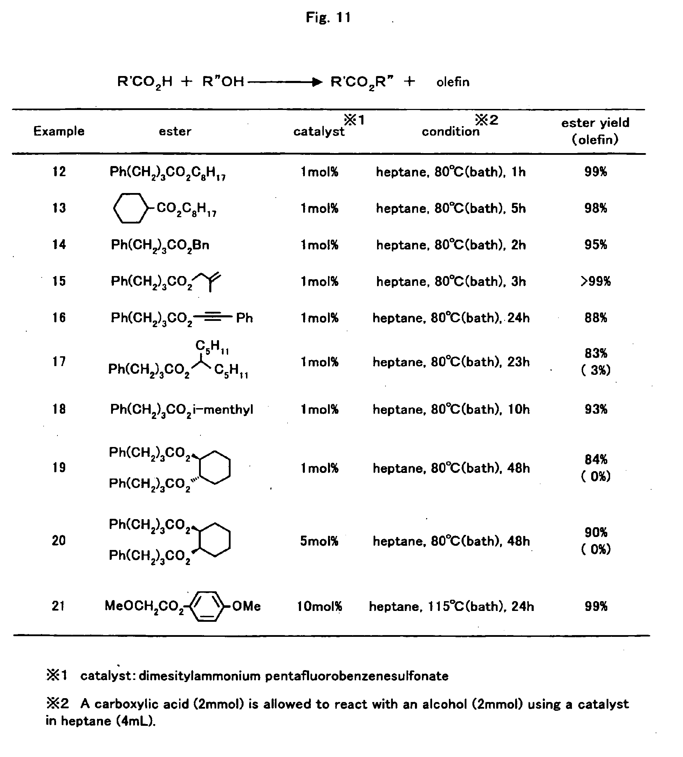

An esterification reaction in each of Examples 12 to 21 was carried out under the conditions shown in the table of Fig. 11. As a result, the esterification reaction proceeded with the yield shown in the same table. As is evident from the table, even when the alcohol that was susceptible to acids, such as benzyl alcohol (Example 14) or allyl alcohol (Example 15), was used, the corresponding ester compound was obtained in extremely high yield. Furthermore, even when 1,2-diol (Examples 19 and 20), which was not able to be used with a Lewis acid metal, such as a hafnium (IV) salt or a zirconium (IV) salt, was used, the corresponding ester compounds were obtained in high yield.

### [Examples 22 to 27]

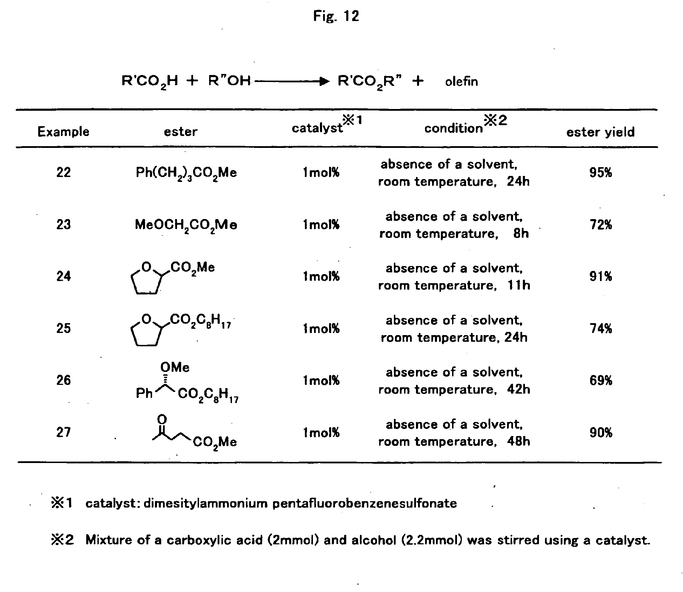

An esterification reaction in each of Examples 22 to 27 was carried out under the conditions shown in the table of Fig. 12. As a result, the esterification reaction proceeded with the yield shown in the same table. As is evident from the table, when primary alcohols were used, esterification proceeded in high yield even under the conditions of absence of a solvent and room temperature (22°C in this case). In particular, when methanol was used as the primary alcohol (Examples 22 to 24 and 27), good results were obtained.

### [Example 28]

With respect to ester condensation between 4-phenylbutyric acid and 6-undecanol by use of dimesitylammonium pentafluorobenzenesulfonate as a catalyst, a comparison was made between a case where the ester condensation was carried out in hexane without removing produced water and a case where the ester condensation was carried out while removing produced water by azeotropic dehydration. As a result, the changes with time in the conversion ratio of the alcohol, the ester, and the olefin proceeded in completely the same manner. Thus, it is known that the ester condensation is not affected by water.

### [Example 29]

Two ester condensation reactions between 4-phenylbutyric acid and 6-undecanol were carried out under the same conditions except that in one dimesitylammonium pentafluorobenzenesulfonate was used as the catalyst and in the other dimesitylammonium-p-toluenesulfonate was used as the catalyst. As a result, both showed equivalent catalytic activity.

It is to be understood that the present invention is not limited to the examples described above and numerous variations and modifications can be made without departing the technical scope of the present invention.

### Industrial Applicability

The present invention is mainly applicable to chemical industry. For example, the present invention can be used when various oils, fats, and polyesters are produced and when various carboxylate esters, which are used as intermediates for pharmaceuticals and agrochemicals, are produced.

## Claims

1. An esterification catalyst comprising, as a main component, an ammonium sulfonate composed of an ammonium cation having a basic skeleton represented by formula (1) below, wherein n represents 1 or 2, in which hydrogen atoms in the benzene rings and hydrogen atoms bonded to bridge carbon may be substituted, and a sulfonic acid anion represented by RSO₃, wherein R represents a perfluoroalkane having 1 to 8 carbon atoms, or a benzene having an electron-withdrawing group at least at the ortho position, the meta position or the para position.

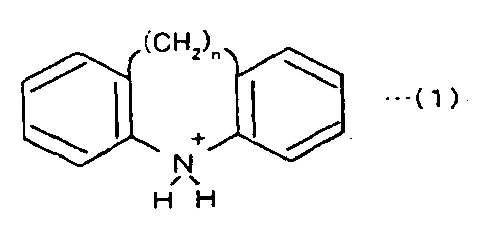

2. The esterification catalyst according to Claim 1, wherein the ammonium cation has the basic skeleton represented by formula (1), wherein n represents 1 or 2, in which the hydrogen atom at the ortho position, the meta position, or the para position of the benzene ring may be substituted with a linear or branched alkyl group having 1 to 10 carbon atoms.

3. The esterification catalyst according to Claim 1 or 2, wherein the ammonium cation is iminodibenzyl represented by formula (1), wherein n represents 2, in which hydrogen atoms in the benzene rings and hydrogen atoms bonded to bridge carbon are not substituted.

4. An esterification catalyst comprising, as a main component, an ammonium sulfonate composed of an ammonium cation having a basic skeleton represented by formula (2) below, wherein R¹ to R⁴ each independently represent a linear or branched alkyl group having 1 to 10 carbon atoms, in which hydrogen atoms in the benzene rings other than R¹ to R⁴ may be substituted, and a sulfonic acid anion represented by RSO₃, wherein R represents a perfluoroalkane having 1 to 8 carbon atoms, or a benzene having an electron-withdrawing group at least at the ortho position, the meta position or the para position.

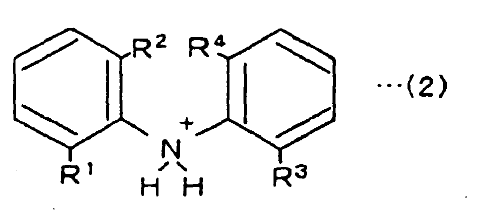

5. The esterification catalyst according to Claim 4, wherein the ammonium cation has the basic skeleton represented by formula (2) in which the hydrogen atom at the ortho position, the meta position, or the para position of the benzene ring is substituted with a linear or branched alkyl group having 1 to 10 carbon atoms.

6. The esterification catalyst according to any one of Claims 1 to 5, wherein the sulfonic acid anion is a trifluoromethane sulfonic acid anion or a pentafluorobenzene sulfonic acid anion.

7. A process for producing an ester comprising the step of allowing a carboxylic acid to react with an alcohol, wherein an esterification catalyst according to any one of claims 1 to 6 is used as an esterification catalyst.

8. The process for producing the ester according to Claim 7, wherein equimolar amounts of the carboxylic acid and the alcohol are used.

9. The process for producing the ester according to Claim 7 or 8, wherein an aliphatic hydrocarbon solvent or an aromatic hydrocarbon solvent is used as a reaction solvent.

10. The process for producing the ester according to any one of Claims 7 to 9, wherein the esterification catalyst is used in an amount of 0.1 to 20 mol% relative to the amount of the carboxylic acid.

11. The process for producing the ester according to any one of Claims 7 to 10, wherein the reaction temperature is set in a range of room temperature to 90°C.

12. The process for producing the ester according to any one of Claims 7 to 10, wherein a primary alcohol is used as the alcohol, no reaction solvent is used, and the reaction temperature is set at room temperature.

13. The process for producing the ester according to any one of Claims 7 to 12, wherein the reaction is allowed to proceed without removing produced water from the reaction system.

## Patentansprüche

1. Veresterungskatalysator, welcher als eine Hauptkomponente ein Ammoniumsulfonat umfasst, das aus einem Ammonium-Kation mit einem Grundgerüst der Formel (1), wobei n für 1 oder 2 steht und in welchem Wasserstoffatome der Benzolringe und Wasserstoffatome, die an einen Brückenkohlenstoff gebunden sind, ersetzt sein können, und einem Sulfonsäure-Anion RSO₃ zusammengesetzt ist, wobei R für ein Perfluoralkan mit 1 bis 8 Kohlenstoffatomen oder für ein Benzol mit einer elektronenziehenden Gruppe zumindest in der Ortho-Position, der Meta-Position oder der Para-Position steht.

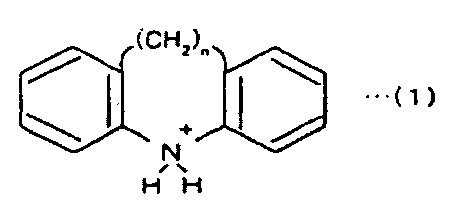

2. Veresterungskatalysator nach Anspruch 1, wobei das Ammonium-Kation das Grundgerüst der Formel (1) aufweist, wobei n für 1 oder 2 steht und in welchem das Wasserstoffatom in der Ortho-Position, der Meta-Position oder der Para-Position des Benzolrings durch eine lineare oder verzweigte Alkylgruppe mit 1 bis 10 Kohlenstoffatomen ersetzt ist.

3. Veresterungskatalysator nach Anspruch 1 oder 2, wobei es sich bei dem Ammonium-Kation um ein Iminodibenzyl-Kation gemäß der Formel (1) handelt, wobei n für 2 steht und wobei die Wasserstoffatome der Benzolringe und die Wasserstoffatome, die an ein Brückenkohlenstoffatom gebunden sind, nicht ersetzt sind.

4. Veresterungskatalysator, welcher als eine Hauptkomponente ein Ammoniumsulfonat umfasst, das aus einem Ammonium-Kation mit einem Grundgerüst der Formel (2), wobei R¹ bis R⁴ jeweils unabhängig voneinander für eine lineare oder verzweigte Alkylgruppe mit 1 bis 10 Kohlenstoffatomen stehen und in welchem die Wasserstoffatome der Benzolringe über R¹ bis R⁴ hinaus ersetzt sein können, und einem Sulfonsäure-Anion RSO₃ zusammengesetzt ist, wobei R für ein Perfluoralkan mit 1 bis 8 Kohlenstoffatomen oder für ein Benzol mit einer elektronenziehenden Gruppe zumindest in der Ortho-Position, der Meta-Position oder der Para-Position steht.

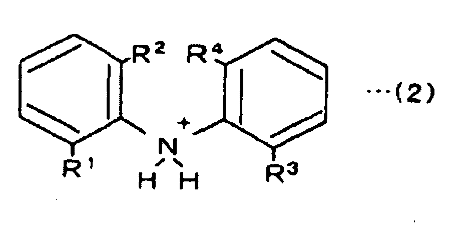

5. Veresterungskatalysator nach Anspruch 4, wobei das Ammonium-Kation das Grundgerüst der Formel (2) aufweist, in welchem das Wasserstoffatom in der Ortho-Position, der Meta-Position oder der Para-Position des Benzolrings durch eine lineare oder verzweigte Alkylgruppe mit 1 bis 10 Kohlenstoffatomen ersetzt ist.

6. Veresterungskatalysator nach einem der Ansprüche 1 bis 5, wobei es sich bei dem Sulfonsäure-Anion um ein Trifluormethansulfonsäure-Anion oder ein Pentafluorbenzolsulfonsäure-Anion handelt.

7. Verfahren zur Herstellung eines Esters, welches den Schritt des Ermöglichens der Reaktion einer Carbonsäure mit einem Alkohol umfasst, wobei als Veresterungskatalysator ein Veresterungskatalysator nach einem der Ansprüche 1 bis 6 verwendet wird.

8. Verfahren zur Herstellung eines Esters nach Anspruch 7, wobei äquimolare Mengen der Carbonsäure und des Alkohols verwendet werden.

9. Verfahren zur Herstellung eines Esters nach Anspruch 7 oder 8, wobei als Lösungsmittel für die Reaktion ein aliphatischer Kohlenwasserstoff oder ein aromatischer Kohlenwasserstoff Lösungsmittel verwendet wird.

10. Verfahren zur Herstellung eines Esters nach einem der Ansprüche 7 bis 9, wobei der Veresterungskatalysator in einer Menge von 0,1 bis 20 Mol-%, bezogen auf die Menge der Carbonsäure, verwendet wird.

11. Verfahren zur Herstellung eines Esters nach einem der Ansprüche 7 bis 10, wobei die Reaktionstemperatur auf eine Temperatur in einem Bereich von Raumtemperatur bis 90°C eingestellt wird.

12. Verfahren zur Herstellung eines Esters nach einem der Ansprüche 7 bis 10, wobei als Alkohol ein primärer Alkohol verwendet wird, kein Reaktionslösungsmittel verwendet wird und die Reaktionstemperatur auf Raumtemperatur eingestellt wird.

13. Verfahren zur Herstellung eines Esters nach einem der Ansprüche 7 bis 12, wobei man die Reaktion voranschreiten lässt, ohne das erzeugte Wasser aus dem Reaktionssystem zu entfernen.

## Revendications

1. Catalyseur d'estérification comprenant, comme composant principal, un sulfonate d'ammonium constitué d'un cation ammonium ayant un squelette de base représenté par la formule (1) ci-dessous, où n vaut 1 ou 2, dans lequelle les atomes d'hydrogène dans les cycles benzène ou les atomes d'hydrogène liés au pont carbone peuvent être substitués, et un anion acide sulfonique représenté par RSO₃, où R représente un perfluoroalcane ayant 1 à 8 atomes de carbone, ou un benzène ayant un groupe attracteur d'électrons au moins en position ortho, méta ou para.

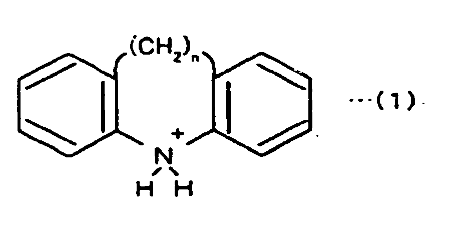

2. Catalyseur d'estérification selon la revendication 1, dans lequel le cation ammonium a le squelette de base représenté par la formule (1), où n vaut 1 ou 2, dans lequel l'atome d'hydrogène en position ortho, méta ou para du cycle benzène peut être substitué par un groupe alkyle linéaire ou ramifié ayant 1 à 10 atomes de carbone.

3. Catalyseur d'estérification selon la revendication 1 ou 2, dans lequel le cation ammonium est un iminodibenzyle représenté par la formule (1), où n vaut 2, dans lequel les atomes d'hydrogène dans les cycles benzène et les atomes d'hydrogène liés au pont carbone ne sont pas substitués.

4. Catalyseur d'estérification comprenant, comme composant principal, un sulfonate d'ammonium constitué d'un cation ammonium ayant un squelette de base représenté par la formule (2) ci-dessous, où R¹ à R⁴ représentent chacun indépendamment les uns des autres un groupe alkyle linéaire ou ramifié ayant 1 à 10 atomes de carbone, dans lequel les atomes d'hydrogène dans les cycles benzène autres que R¹ à R⁴ peuvent être substitués, et un anion acide sulfonique représenté par RSO₃, où R représente un perfluoroalcane ayant 1 à 8 atomes de carbone, ou un benzène ayant un groupe attracteur d'électrons au moins en position ortho, méta ou para.

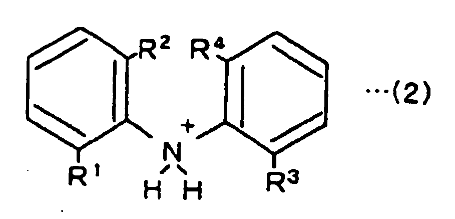

5. Catalyseur d'estérification selon la revendication 4, dans lequel le cation ammonium a le squelette de base représenté par la formule (2), dans lequel l'atome d'hydrogène en position ortho, méta ou para du cycle benzène est substitué par un groupe alkyle linéaire ou ramifié ayant 1 à 10 atomes de carbone.

6. Catalyseur d'estérification selon l'une quelconque des revendications 1 à 5, dans lequel l'anion acide sulfonique est un anion acide sulfonique de trifluorométhane ou un anion acide sulfonique de pentafluorobenzène.

7. Procédé de production d'un ester comprenant l'étape permettant à un acide carboxylique de réagir avec un alcool, dans lequel un catalyseur d'estérification selon l'une quelconque des revendications 1 à 6 est utilisé comme catalyseur d'estérification.

8. Procédé de production de l'ester selon la revendication 7, dans lequel des quantités équimolaires d'acide carboxylique et d'alcool sont utilisées.

9. Procédé de production de l'ester selon la revendication 7 ou 8, dans lequel un solvant hydrocarbure aliphatique ou un solvant hydrocarbure aromatique est utilisé comme solvant de réaction.

10. Procédé de production de l'ester selon l'une quelconque des revendications 7 à 9, dans lequel le catalyseur d'estérification est utilisé dans une quantité de 0,1 à 20 % en mole par rapport à la quantité d'acide carboxylique.

11. Procédé de production de l'ester selon l'une quelconque des revendications 7 à 10, dans lequel la température de réaction est réglée entre la température ambiante et 90°C.

12. Procédé de production de l'ester selon l'une quelconque des revendications 7 à 10, dans lequel un alcool primaire est utilisé comme alcool, aucun solvant de réaction n'est utilisé et la température de réaction est réglée au niveau de la température ambiante.

13. Procédé de production de l'ester selon l'une quelconque des revendications 7 à 12, dans lequel la réaction est effectuée sans éliminer l'eau produite du système de réaction.
